(19) Europäisches Patentamt
European Patent Office
Office européen des brevets

(11) Veröffentlichungsnummer: **0 080 435**
**A2**

# (12) EUROPÄISCHE PATENTANMELDUNG

(21) Anmeldenummer: **82810496.8**

(22) Anmeldetag: **19.11.82**

(51) Int. Cl.³: **C 07 D 239/47**, A 01 N 47/18

(30) Priorität: **25.11.81 CH 7537/81**
**26.10.82 CH 6237/82**

(43) Veröffentlichungstag der Anmeldung: **01.06.83**
**Patentblatt 83/22**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Hoegerle, Karl, Dr., Reinacherstrasse 68, CH-4053 Basel (CH)**
Erfinder: **Gsell, Laurenz, Dr., Maiengasse 56, CH-4056 Basel (CH)**
Erfinder: **Wehrli, Rudolf, Dr., Dianastrasse 2, CH-4310 Rheinfelden (CH)**

(54) **4-N,N-Dimethylcarbamoyloxy-6-aminopyrimidine und ihre Salze, Verfahren zu ihrer Herstellung und ihre Verwendung in der Schädlingsbekämpfung.**

(57) 4-N,N-Dimethylcarbamoyloxy-6-aminopyrimidine und ihre Salze der Formel

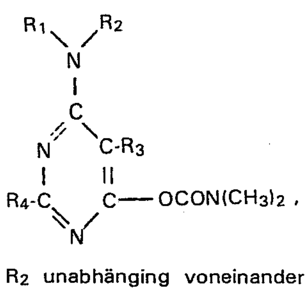

worin $R_1$ und $R_2$ unabhänging voneinander Wasserstoff, $C_1$-$C_5$-Alkyl, $C_1$-$C_5$-Halogenalkyl, $C_3$-$C_6$-Cycloalkyl, $C_2$-$C_5$-Alkenyl, $C_2$-$C_5$-Alkinyl oder zusammen einen $C_2$-$C_5$-Alkykenrest, $R_3$ Halogen oder $C_1$-$C_5$-Alkyl und $R_4$ $C_1$-$C_5$-Alkyl oder $R_3$ Wasserstoff und $R_4$ $C_2$-$C_5$-Alkyl bedeuten,

Ein Verfahren zur Herstellung dieser Pyrimidine, ihre Verwendung in der Schädlingsbekämpfung sowie die Zwischenprodukte der Formel

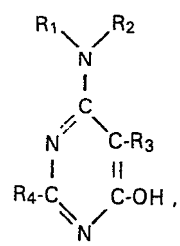

worin $R_1$ und $R_2$ unabhänging voneinander Wasserstoff, $C_1$-$C_5$-Alkyl, $C_1$-$C_5$-Halogenalkyl, $C_3$-$C_6$-Cycloalkyl, $C_2$-$C_5$-Alkenyl, $C_2$-$C_5$-Alkinyl oder zusammen einen $C_2$-$C_5$-Alkylenrest, $R_3$ Halogen oder $C_1$-$C_5$-Alkyl und $R_4$ $C_1$-$C_5$-Alkyil oder $R_3$ Wasserstoff und $R_4$ $C_2$-$C_5$-Alkyl bedeuten, werden beschrieben.

EP 0 080 435 A2

5-13683/1+2

4-N,N-Dimethylcarbamoyloxy-6-aminopyrimidine und ihre Salze, Verfahren zu ihrer Herstellung und ihre Verwendung in der Schädlingsbekämpfung.

Die vorliegende Erfindung betrifft 4-N,N-Dimethylcarbamoyloxy-6-aminopyrimidine und ihre Salze, Verfahren zu ihrer Herstellung und ihre Verwendung in der Schädlingsbekämpfung.

Die 4-N,N-Dimethylcarbamoyloxy-6-aminopyrimidine haben die Formel

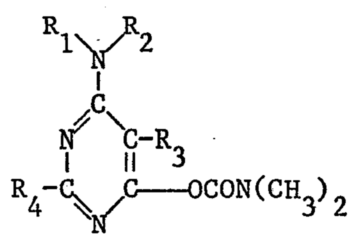

(I) ,

worin $R_1$ und $R_2$ unabhängig voneinander Wasserstoff, $C_1$-$C_5$-Alkyl, $C_1$-$C_5$-Halogenalkyl, $C_3$-$C_6$-Cycloalkyl, $C_2$-$C_5$-Alkenyl, $C_2$-$C_5$-Alkinyl oder zusammen einen $C_2$-$C_5$-Alkylenrest, $R_3$ Halogen oder $C_1$-$C_5$-Alkyl und $R_4$ $C_1$-$C_5$-Alkyl oder $R_3$ Wasserstoff und $R_4$ $C_2$-$C_5$-Alkyl bedeuten.

Für die Salzbildung kommen anorganische Säuren wie beispielsweise HCl, $H_2SO_4$, HBr und $H_3PO_4$ und organische Säuren, beispielsweise gesättigte und ungesättigte Mono-, Di- und Tricarbonsäuren wie z.B. Ameisensäure, Essigsäure, Oxalsäure, Phthalsäure, Bernsteinsäure und Zitronensäure in Betracht. Unter Halogen bei $R_3$ ist Fluor, Chlor, Brom oder Jod, insbesondere aber Chlor oder Brom, zu verstehen.

Die bei $R_1$ bis $R_4$ in Frage kommenden Alkyl-, Halogenalkyl-, Alkenyl- oder Alkinylgruppen können geradkettig oder verzweigt sein. Beispiele solcher Gruppen sind u.a.: Methyl, Trifluormethyl, Aethyl, $-CH_2CHF_2$,

Propyl, Isopropyl, n-, i-, sek.-, tert.-Butyl, n-Pentyl und dessen Isomere, 1-Propenyl, 2-Propenyl und 1-Propinyl. Beispiele von Cycyloalkylgruppen bei $R_1$ und $R_2$ sind Cyclopropyl, Cyclopentyl und Cyclohexyl, vorzugsweise Cyclopropyl.

Bevorzugt sind Verbindungen der Formel I, worin $R_1$ und $R_2$ unabhängig voneinander Wasserstoff, $C_1$-$C_5$-Alkyl, Cyclopropyl, 1-Propenyl, 2-Propenyl oder 1-Propinyl oder $R_1$ und $R_2$ zusammen mit dem Stickstoffatom, an das sie gebunden sind, den Pyrrolidinring bilden, $R_3$ Chlor, Brom oder Methyl und $R_4$ Methyl, Aethyl oder Isopropyl oder $R_3$ Wasserstoff und $R_4$ Isopropyl bedeuten.

Insbesondere bevorzugt sind Verbindungen der Formel I, worin $R_1$ und $R_2$ unabhängig voneinander Wasserstoff, $C_1$-$C_5$-Alkyl oder 2-Propenyl, $R_3$ Chlor, Brom oder Methyl und $R_4$ Methyl, Aethyl oder Isopropyl oder $R_3$ Wasserstoff und $R_4$ Isopropyl bedeuten.

Die Verbindungen der Formel I werden nach an sich bekannten Methoden z.B. wie folgt hergestellt:

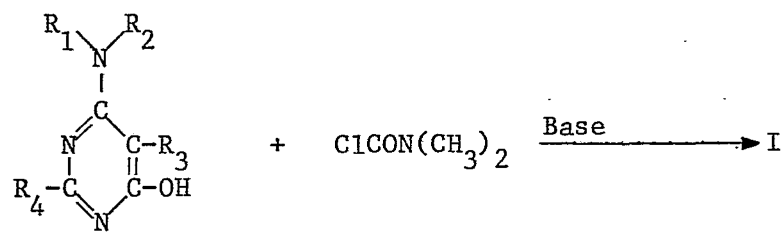


(II)

In der Formel II haben $R_1$ bis $R_4$ die für die Formel I angegebene Bedeutung.

Als geeignete Basen kommen insbesondere tertiäre Amine wie Trialkylamine, Dialkylaniline und p-Dialkylaminopyridine in Betracht.

Das Verfahren wird bei normalem Druck, bei einer Temperatur von -25° bis 150°C, vorzugsweise bei 50° bis 100°C und gegebenenfalls in einem Lösungs- oder Verdünnungsmittel durchgeführt.

Als Lösungs- oder Verdünnungsmittel eignen sich z.B. Aether und ätherartige Verbindungen wie Diäthyläther, Diisopropyläther, Dioxan und Tetrahydrofuran; aromatische Kohlenwasserstoffe wie Benzol, Toluol und Xylole; Ketone wie Aceton, Methyläthylketon und Cyclohexanon; Nitrile wie Acetonitril; Ester wie Aethylacetat und Butylacetat; sowie Dimethylformamid, Dimethylsulfoxid, Methylcyanid und halogenierte Kohlenwasserstoffe.

Die Ausgangsstoffe der Formel II sind neu und bilden ebenfalls einen Gegenstand der vorliegenden Erfindung; sie können aber nach bekannten Verfahren hergestellt werden z.B. durch Reaktion einer Verbindung der Formel

(III) 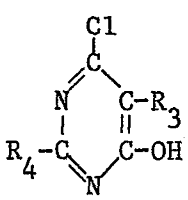 [vgl. J. Org. Chem. 34, 2972 (1969)]

mit einer Verbindung der

Formel 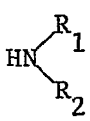 gegebenenfalls in Gegenwart eines Lösungsmittels und

(IV)

einer Base wie z.B. Natronlauge.

In den Formeln III und IV haben $R_1$ bis $R_4$ die für die Formel I angegebene Bedeutung.

Die Verbindungen der Formel I eignen sich zur Bekämpfung von Schädlingen an Tieren und Pflanzen. Ferner haben diese Verbindungen auch fungizide und pflanzenregulatorische Eigenschaften.

Insbesondere eignen sich die Verbindungen der Formel I zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Akarina.

Vor allem eignen sich Verbindungen der Formel I zur Bekämpfung von pflanzenschädigenden Insekten, insbesondere pflanzenschädigenden, saugenden Insekten, in Zier- und Nutzpflanzen, insbesondere in Baumwollkulturen, Gemüsekulturen, Reiskulturen und Obstkulturen.

Wirkstoffe der Formel I zeigen auch eine günstige Wirkung gegen fressende und beissende Insekten sowie gegen Fliegen wie z.B. Musca domestica und Mückenlarven.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Aethylenglykoleinheiten.

Als Beispiele nichtionischer Tenside seien Nonylphenolpolyäthoxyäthanole, Ricinusölpolyglykoläther, Polypropylenpolyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi-(2-chloräthyl)-äthylammoniumbromid.

Die in der Formulierungstechnik gebräuchlichen Tenside sind u.a. in folgender Publikation beschrieben:

"Mc Cutcheon's Detergents and Emulsifiers Annual" MC Publishing Corp., Ringwood, New Jersey, 1979.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I (% = Gewichtsprozent)

| 1. Emulsions-Konzentrate | a) | b) | c) |
|---|---|---|---|
| Wirkstoff | 25% | 40% | 50% |
| Ca-Dodecylbenzolsulfonat | 5% | 8% | 6% |
| Ricinusölpolyäthylenglykoläther (36 Mol AeO) | 5% | - | - |
| Tributylphenolpolyäthylenglykoläther (30 Mol AeO) | - | 12% | 4% |
| Cyclohexanon | - | 15% | 20% |
| Xylolgemisch | 65% | 25% | 20% |

Aus solche Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol MG 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| 3. Granulate | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
| --- | --- | --- |
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

| 5. Spritzpulver | a) | b) | c) |
| --- | --- | --- | --- |
| Wirkstoff | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykol-äther (7-8 Mol AeO) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
| --- | --- |
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

| 8. Extruder Granulat | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| 9. Umhüllungs-Granulat | | |
|---|---|---|
| Wirkstoff | | 3% |
| Polyäthylenglykol | (MG 200) | 3% |
| Kaolin | | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40% |
| Aethylenglykol | 10% |
| Nonylphenolpolyäthylenglykol-äther (15 Mol AeO) | 6 % |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1:

a) Herstellung der Verbindung der Formel

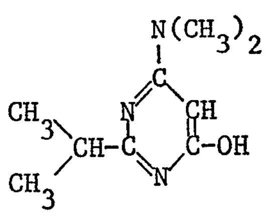

58 g 2-Isopropyl-4-chlor-6-hydroxypyrimidin-hydrochlorid [J.Org.Chem. 34, 2972 (1969)] und 100 ml alkoholische Dimethylamin-Lösung (33%) werden 10 Stunden auf 100°C erwärmt. Nach dem Erkalten wird die feinkörnige Suspension filtriert und der Rückstand in 800 ml Wasser angerührt und mit konz. Natronlauge auf pH 10-11 gestellt. Die erwärmte Suspension wird filtriert und das Filtrat mit Essigsäure auf pH 6 gestellt. Nach dem Abkühlen werden die ausgefallenen Kristalle abfiltriert und aus Methylalkohol umkristallisiert. Smp. 196-197°C.

b) Herstellung der Verbindung Nr. 1

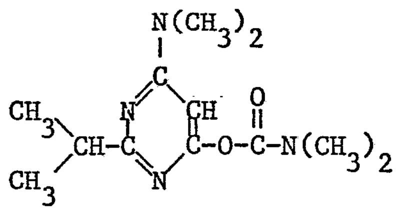

13,6 g 2-Isopropyl-4-dimethylamino-6-hydroxy-pyrimidin, 10 g Dimethylcarbamoylchlorid, 9,5 g Triäthylamin, 0,2 g Dimethylamino-pyridin in 150 ml Chloroform werden 20 Stunden lang am Rückfluss gehalten. Nach dem Abfiltrieren des unlöslichen Anteils und dem Entfernen des Lösungsmittels wird das Rohprodukt in Aether/Pentan umkristallisiert. Smp. 60-61°C.

Auf analoge Weise werden auch folgende Verbindungen hergestellt:

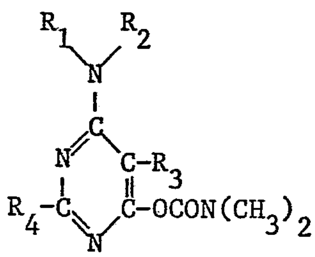

| Nr. | $R_1$ | $R_2$ | $R_3$ | $R_4$ | Physikalische Daten |
|---|---|---|---|---|---|
| 2 | $-CH_2CH=CH_2$ | H | $-CH_3$ | $-C_2H_5$ | Smp.: 62-64°C |
| 3 | $-CH_3$ | $-CH_3$ | -Br | $-CH_3$ | $n_D^{20°}$ = 1,5443 |
| 4 | $-CH_3$ | $-CH_3$ | -Br | $-C_3H_{7(i)}$ | $n_D^{20°}$ = 1,5265 |
| 5 | $-CH_3$ | $-CH_3$ | $-CH_3$ | $-C_2H_5$ | $n_D^{20°}$ = 1,5270 |
| 6 | $-CH_2CH=CH_2$ | H | H | $-C_3H_{7(i)}$ | $n_D^{20°}$ = 1,5212 |

| Nr. | $R_1$ | $R_2$ | $R_3$ | $R_4$ | Physikalische Daten |
|---|---|---|---|---|---|
| 7 | $-CH_3$ | $-CH_3$ | $-C_2H_5$ | n-$C_3H_7$ | $n_D^{20°}$ = 1,5118 |
| 8 | $-CH_3$ | H | $-CH_3$ | $-C_2H_5$ | Smp.: 145-146°C |
| 9 | $-C_2H_5$ | H | $-CH_3$ | $-C_2H_5$ | Smp.: 91-94°C |
| 10 | H | H | $-CH_3$ | $-C_2H_5$ | Smp.: 138-144°C |
| 11 | $-C_2H_5$ | H | $-C_2H_5$ | n-$C_3H_7$ | Smp.: 73-75°C |
| 12 | H | H | $-C_2H_5$ | n-$C_3H_7$ | Smp.: 54-55°C |
| 13 | $-CH_3$ | $-CH_3$ | $-CH_3$ | $-C_2H_5$ | Smp.: 68-69°C |

Beispiel 2: Insektizide Kontaktwirkung: Aphis craccivora

In Töpfen angezogene Pflanzen (Vicia fabae) werden vor dem Versuchsbeginn mit je ca. 200 Individuen der Spezies Aphis fabae besiedelt. Die so behandelten Pflanzen werden 24 Stunden später mit einer Lösung enthaltend 3, 12,5 , 50 oder 100 ppm der zu prüfenden Verbindung bis zur Tropfnässe besprüht. Man verwendete pro Test-Verbindung und pro Konzentration zwei Pflanzen, und eine Auswertung der erzielten Abtötungsrate erfolgt nach weiteren 24 Stunden.

Die Verbindungen gemäss Beispiel 1 zeigen im obigen Versuch die in der folgenden Tabelle angegebene Wirkung gegen Insekten der Spezies Aphis craccivora.

Beispiel 3: Insektizide Wirkung (systemisch): Aphis craccivora

Bewurzelte Bohnenpflanzen werden in Töpfe, welche 600 ccm Erde enthalten, verpflanzt und anschliessend 50 ml einer Lösung der zu prüfenden Verbindung (erhalten aus einem 25%igen Spritzpulver) in einer Konzentration von 3, 12,5 , 50 oder 100 ppm direkt auf die Erde aufgegossen.

Nach 24 Stunden werden auf die oberirdischen Pflanzenteile Läuse der Spezies Aphis craccivora gesetzt und die Pflanzen mit einem Plastikzylinder überstülpt, um die Läuse vor einer eventuellen Kontakt- oder Gaswirkung der Testsubstanz zu schützen.

Die Auswertung der erzielten Abtötung erfolgt 48 und 72 Stunden nach Versuchsbeginn. Pro Konzentrationsdosis Testsubstanz wird zwei Pflanzen, je eine in einem separaten Topf, verwendet. Der Versuch wird bei 25°C und 70% relativer Luftfeuchtigkeit durchgeführt.

Die Verbindungen gemäss Beispiel 1 zeigen im obigen Versuch die in der folgenden Tabelle angegebene systemische Wirkung gegen Insekten der Spezies Aphis craccivora.

Biologische Versuchsergebnisse

In der folgenden Tabelle sind Versuchsergebnisse auf der Basis der vorstehenden Beispiele aufgeführt, und zwar mit folgendem Bewertungsindex in Bezug auf die prozentuale Abtötung der Schädlinge.

A : 70-100% Abtötung bei 3 ppm Wirkstoffkonzentration.

B : 70-100% Abtötung bei 12,5 ppm Wirkstoffkonzentration.

C : 70-100% Abtötung bei 50 ppm Wirkstoffkonzentration.

D : 70-100% Abtötung bei 100 ppm Wirkstoffkonzentration.

| Verb. Nr. | Kontakt Wirkung gegen Aphis craccivora | Systemische Wirkung gegen Aphis craccivora |
|---|---|---|
| 1 | B | C |
| 2 | A | A |
| 3 | A | A |
| 4 | C | D |
| 5 | A | A |
| 6 | D | D |
| 7 | B | A |
| 8 | C | A |
| 9 | B | A |
| 10 | B | A |
| 11 | B | A |
| 12 | B | A |
| 13 | B | A |

Patentansprüche

1. Ein 4-N,N-Dimethylcarbamoyloxy-6-aminopyrimidin und dessen Salze der Formel

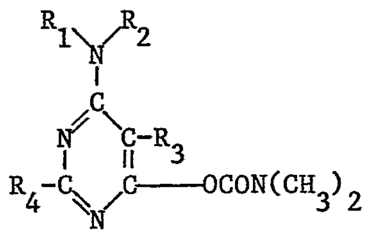

(I) ,

worin $R_1$ und $R_2$ unabhängig voneinander Wasserstoff, $C_1$-$C_5$-Alkyl, $C_1$-$C_5$-Halogenalkyl, $C_3$-$C_6$-Cycloalkyl, $C_2$-$C_5$-Alkenyl, $C_2$-$C_5$-Alkinyl oder zusammen einen $C_2$-$C_5$-Alkylenrest, $R_3$ Halogen oder $C_1$-$C_5$-Alkyl und $R_4$ $C_1$-$C_5$-Alkyl oder $R_3$ Wasserstoff und $R_4$ $C_2$-$C_5$-Alkyl bedeuten.

2. Eine Verbindung gemäss Anspruch 1, worin $R_1$ und $R_2$ unabhängig voneinander Wasserstoff, $C_1$-$C_5$-Alkyl, Cyclopropyl, 1-Propenyl, 2-Propenyl, oder 1-Propinyl oder $R_1$ und $R_2$ zusammen mit dem Stickstoffatom, an das sie gebunden sind, den Pyrrolidinring bilden, $R_3$ Chlor, Brom oder Methyl und $R_4$ Methyl, Aethyl oder Isopropyl oder $R_3$ Wasserstoff und $R_4$ Isopropyl bedeuten.

3. Eine Verbindung gemäss Anspruch 2, worin $R_1$ und $R_2$ unabhängig voneinander Wasserstoff, $C_1$-$C_5$-Alkyl oder 2-Propenyl, $R_3$ Chlor, Brom oder Methyl und $R_4$ Methyl, Aethyl oder Isopropyl oder $R_3$ Wasserstoff und $R_4$ Isopropyl bedeuten.

4. Die Verbindung gemäss Anspruch 3 der Formel

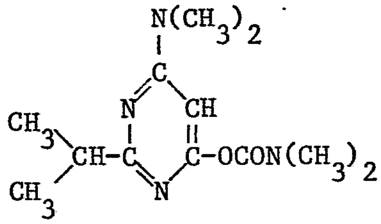

.

5. Die Verbindung gemäss Anspruch 3 der Formel

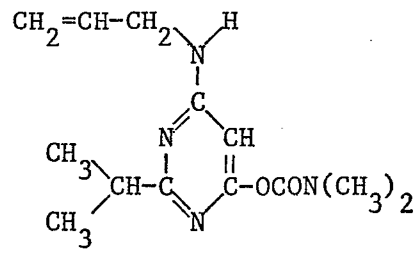 .

6. Die Verbindung gemäss Anspruch 3 der Formel

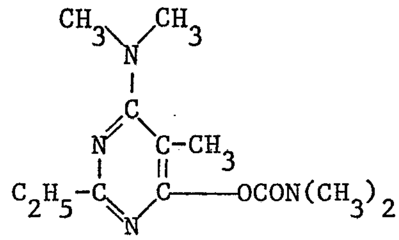 .

7. Die Verbindung gemäss Anspruch 3 der Formel

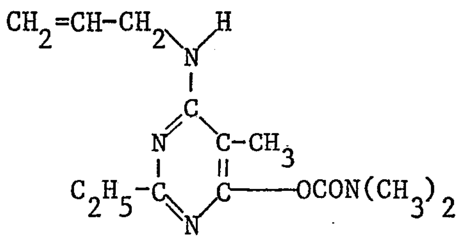 .

8. Die Verbindung gemäss Anspruch 3 der Formel

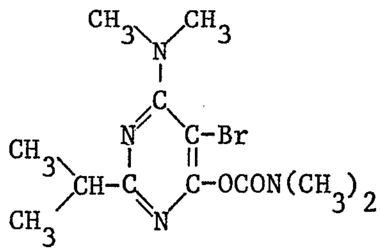 .

9. Die Verbindung gemäss Anspruch 3 der Formel

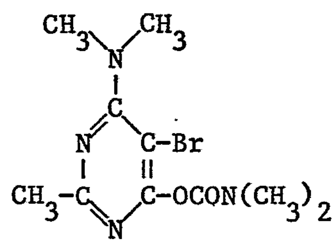

.

10. Die Verbindung gemäss Anspruch 3 der Formel

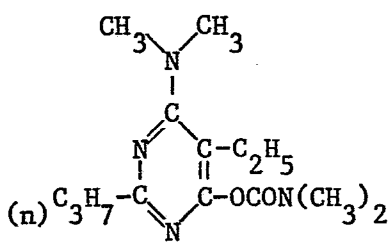

.

11. Die Verbindung gemäss Anspruch 3 der Formel

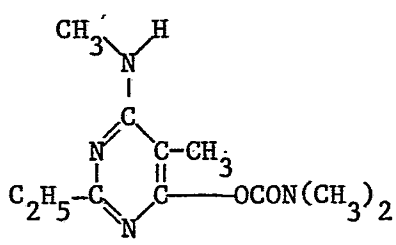

.

12. Die Verbindung gemäss Anspruch 3 der Formel

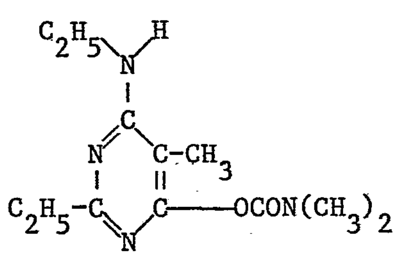

.

13. Die Verbindung gemäss Anspruch 3 der Formel

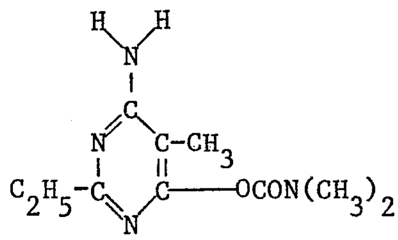

.

14. Die Verbindung gemäss Anspruch 3 der Formel

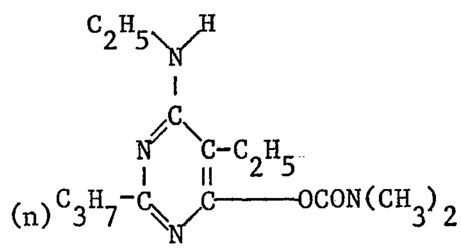

.

15. Die Verbindung gemäss Anspruch 3 der Formel

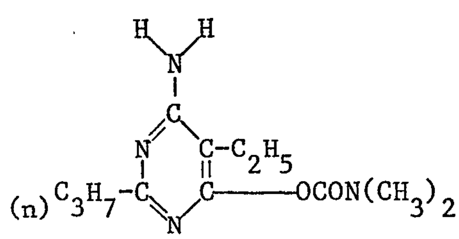

.

16. Die Verbindung gemäss Anspruch 3 der Formel

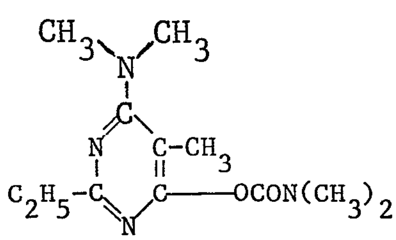

.

17. Ein Verfahren zur Herstellung von Verbindungen gemäss Anspruch 1, dadurch gekennzeichnet, dass man eine Verbindung der Formel

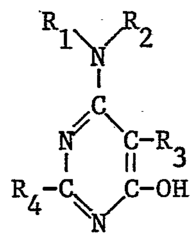

in Gegenwart einer Base mit Dimethylcarbamoylchlorid umsetzt, worin $R_1$ bis $R_4$ die im Anspruch 1 angegebene Bedeutung haben.

18. Ein Schädlingsbekämpfungsmittel, welches als aktive Komponente eine Verbindung gemäss Anspruch 1 und geeignete Träger- und/oder andere Zuschlagstoffe enthält.

19. Verwendung einer Verbindung gemäss Anspruch 1 zur Bekämpfung von Schädlingen an Tieren und Pflanzen.

20. Verwendung gemäss Anspruch 19 zur Bekämpfung von Insekten und Vertretern der Ordnung Akarina.

21. Eine Verbindung der Formel

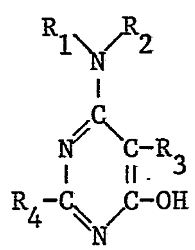 ,

worin $R_1$ und $R_2$ unabhängig voneinander Wasserstoff, $C_1$-$C_5$-Alkyl, $C_1$-$C_5$-Halogenalkyl, $C_3$-$C_6$-Cycloalkyl, $C_2$-$C_5$-Alkenyl, $C_2$-$C_5$-Alkinyl oder zusammen einen $C_2$-$C_5$-Alkylenrest, $R_3$ Halogen oder $C_1$-$C_5$-Alkyl und $R_4$ $C_1$-$C_5$-Alkyl oder $R_3$ Wasserstoff und $R_4$ $C_2$-$C_5$-Alkyl bedeuten.